# EUROPEAN PATENT APPLICATION

(11) **EP 3 779 437 A1**
(43) Date of publication of application: **17.02.2021**
(21) Application number: 19774766.0
(22) Date of filing: 29.03.2019
(51) Int. Cl.: G01N 33/53, C07K 16/18, C07K 16/24, G01N 33/68

(54) **NOVEL AORTIC ANEURYSM MARKER**

(30) Priority: 30.03.2018 WO PCT/JP2018/013571
(71) Applicant: EIKEN KAGAKU KABUSHIKI KAISHA, Tokyo 110-8408 (JP); National Cerebral and Cardiovascular Center, Suita-shi, Osaka 564-8565 (JP)
(72) Inventor: MINAMINO, Naoto, Suita-shi, Osaka 565-8565 (JP); YAGI, Hiroaki, Suita-shi, Osaka 565-8565 (JP); NISHIGORI, Mitsuhiro, Suita-shi, Osaka 565-8565 (JP); UEDA, Hatsue, Suita-shi, Osaka 565-8565 (JP); MATSUDA, Hitoshi, Suita-shi, Osaka 565-8565 (JP); MURAKAMI, Yusuke, Shimotsuga-gun, Tochigi 329-0114 (JP)
(74) Representative: Altmann Stößel Dick Patentanwälte PartG mbB
(86) International application number: PCT/JP2019/013959
(87) International publication number: WO 2019/189744

(57) **Abstract**

The present invention is intended to provide a novel biomarker capable of detecting aortic aneurysm with high sensitivity, and specifically, the present invention relates to a detection marker of aortic aneurysm, comprising an NPC2 (Niemann-Pick disease type C2) protein and/or an IGFBP7 (Insulin-like growth factor-binding protein 7) protein.

## Description

### Technical Field

The present invention relates to the technical field of a clinical test agent that assists the diagnosis of aortic aneurysm. More specifically, the present invention relates to a biomarker of aortic aneurysm, and an agent for detecting aortic aneurysm, a kit for detecting aortic aneurysm and a method for detecting aortic aneurysm, in each of which the aforementioned biomarker is used.

### Background Art

Aortic aneurysm is a state in which a part of the wall of the aorta is circumferentially or locally diameter-enlarged or projected. Depending on the occurrence site of aneurysm, the aortic aneurysm is referred to as "thoracic aortic aneurysm" in the case of thoracic aorta. It is also referred to as "thoracoabdominal aortic aneurysm" that is generated in some or all of the aorta in both the chest and abdomen, or as "abdominal aortic aneurysm" in the case of abdominal aorta (Non Patent Literature 1). The age of peak incidence of aortic aneurysm is 70s to 80s, and in recent years, the number of patients with aortic aneurysm has increased due to aging of population. However, aortic aneurysm is not discovered, unless it is inspected. This disease progresses with almost no symptoms, and once rupture of aortic aneurysm has occurred, it results in very serious conditions, facing a crisis of life. Since such rupture can be avoided by performing an appropriate treatment, it is extremely important to detect aortic aneurysm in an early stage.

In almost all cases, thoracic aortic aneurysm is found, by chance, when a chest X-ray image is taken in a medical checkup and the like. In the case of abdominal aortic aneurysm, it is often found as a pulsing lump when the abdomen is palpated to diagnose digestive disease such as gastric ulcer or cholelithiasis, or during the abdominal echo (ultrasonic wave) examination of digestive tract, kidney, prostate, etc., or during the MRI examination of the chest and abdomen.

Meanwhile, as markers for aortic aneurysm, D-dimer, CRP, WBC, plasma homocysteine and the like have been known (Non Patent Literatures 2 and 3). However, from the viewpoint of sensitivity, specificity and disease specificity, all of these markers are not considered to be sufficient. Thus, it has been desired to develop a biomarker capable of detecting aortic aneurysm with high sensitivity.

### Citation List

### Non Patent Literature

Non Patent Literature 1: Guidelines regarding Diagnosis and Treatment of Cardiovascular Diseases (Joint working group report, Year 2010), Guidelines for Diagnosis and Treatment of Aortic aneurysm and Aortic Dissection, Revised Edition in 2011
Non Patent Literature 2: Balmforth Damian, et al., General Thoracic and Cardiovascular Surgery, 2017 Oct 28. Doi: 10.1007/s11748-017-0855-0
Non Patent Literature 3: Wanhainen Anders, et al., Arteriosclerosis, Thrombosis, and Vascular Biology, 36(2), 236-44, 2016

### Summary of Invention

### Technical Problem

The present invention has been made under the aforementioned circumstances, and it is an object of the present invention to provide a novel biomarker capable of detecting aortic aneurysm with high sensitivity.

### Solution to Problem

The present inventors have conducted intensive studies for the purpose of providing an aortic aneurysm marker that is excellent in terms of sensitivity and specificity, and have performed proteome analysis using thoracic aortic aneurysm tissues. Factors significantly fluctuating in diseased tissues were obtained by comparing the proteome data of the aortic tissues of healthy subjects and the proteome data of the aortic tissues of patients suffering from thoracic aortic aneurysm, and thereafter, from these factors, a novel marker capable of detecting aortic aneurysm in blood, with high sensitivity and high specificity, was found, thereby completing the present invention.

Specifically, the present invention consists of the following components.
(1) A method for detecting aortic aneurysm, comprising a step of measuring the following 1) and/or 2) in a sample obtained from a subject:
   1) an NPC2 (Niemann-Pick disease type C2) protein, and/or
   2) an IGFBP7 (Insulin-like growth factor-binding protein 7) protein.
(2) The method according to the above (1), further comprising a step of measuring 3) a TSP1 (Thrombospondin 1) protein and/or 4) a PROF1 (Profilin 1) protein.
(3) The method according to the above (1), wherein the subject is an animal in need of prevention or treatment of aortic aneurysm, and the effects of a preventive or therapeutic agent for aortic aneurysm are evaluated or determined by using the NPC2 protein and/or the IGFBP7 protein as indicator(s).
(4) The method according to the above (2), wherein the subject is an animal in need of prevention or treatment of aortic aneurysm, and the effects of a preventive or therapeutic agent for aortic aneurysm are evaluated or determined by using the NPC2 protein and/or the IGFBP7 protein and the TSP1 protein and/or the PROF1 protein as indicators.
(5) A method for detecting or screening a subject having a risk of developing aortic aneurysm in future, comprising a step of measuring the following 1) and/or 2) in a sample obtained from the subject:
   1) an NPC2 (Niemann-Pick disease type C2) protein, and/or
   2) an IGFBP7 (Insulin-like growth factor-binding protein 7) protein.
(6) The method according to the above (5), further comprising a step of measuring 3) a TSP1 (Thrombospondin 1) protein and/or 4) a PROF1 (Profilin 1) protein.
(7) The method according to any one of the above (1) to (6), wherein the aortic aneurysm is thoracic aortic aneurysm, thoracoabdominal aortic aneurysm, and/or abdominal aortic aneurysm.
(8) The method according to any one of the above (1) to (7), wherein the sample is selected from the group consisting of serum, plasma and blood.
(9) The method according to any one of the above (1) to (8), wherein the subject is a human.
(10) The method according to any one of the above (1) to (9), wherein the measurement method is immunoassay or mass spectrometry.
(11) A detection marker of aortic aneurysm, comprising the following 1) and/or 2):
   1) an NPC2 protein, and/or
   2) an IGFBP7protein.
(12) The detection marker according to the above (11), further comprising 3) a TSP1 protein and/or 4) a PROF1 protein.
(13) The detection marker according to the above (11) or (12), wherein the aortic aneurysm is thoracic aortic aneurysm, thoracoabdominal aortic aneurysm, and/or abdominal aortic aneurysm.
(14) An agent for detecting aortic aneurysm, comprising an antibody or an aptamer for measuring the detection marker according to any one of the above (11) to (13).
(15) The detection agent according to the above (14), wherein the antibody is a monoclonal antibody and/or a polyclonal antibody.
(16) A kit for detecting aortic aneurysm, comprising the detection agent according to the above (14) or (15).
(17) The detection marker, detection agent or detection kit according to any one of the above (11) to (16), wherein the sample used in detection of aortic aneurysm is selected from the group consisting of serum, plasma and blood.
(18) Use of the detection marker according to any one of the above (11) to (13), which is for use in screening a preventive or therapeutic agent for aortic aneurysm.
(19) Use of the detection marker according to any one of the above (11) to (13), which is for use in detecting or screening *in vitro* a subject having a risk of developing aortic aneurysm in future.

The present description includes the contents as disclosed in International Application No. PCT/JP2018/013571, which is a priority document of the present application.

### Advantageous Effects of Invention

By using NPC2 or IGFBP7 found by the present invention as a marker of aortic aneurysm, the onset of aortic aneurysm can be detected with high sensitivity and high specificity. Moreover, by using NPC2 and IGFBP7 in combination, aortic aneurysm can be more accurately detected with high sensitivity. Furthermore, by combining NPC2 and/or IGFBP7 with TSP1 and/or PROF1, aortic aneurysm can be detected with high accuracy and high sensitivity.

Further, the agent and kit for detecting aortic aneurysm according to the present invention can be used to assist the early diagnosis of the onset of all of thoracic aortic aneurysm, thoracoabdominal aortic aneurysm, and abdominal aortic aneurysm, and detection of these diseases in an early stage can contribute to the avoidance of an increase in the severity of the diseases. Still further, it is considered that the present agent and kit for detecting aortic aneurysm can also be used in the development and evaluation of a preventive or therapeutic agent for aortic aneurysm, and the present agent and kit can also be utilized in prevention or treatment of aortic aneurysm.

### Brief Description of Drawings

[Figure 1] Figure 1 includes graphs showing the results of the measurement (ELISA method) of NPC2 and IGFBP7 in the serum specimens of thoracic aortic aneurysm patients in Example 1.
[Figure 2] Figure 2 includes graphs showing the ROC (Receiver Operating Characteristic) curves of NPC2, IGFBP7, and the combination thereof in the diagnosis of thoracic aortic aneurysm in Example 1.
[Figure 3] Figure 3 is a graph showing the results of the measurement (ELISA method) of TSP1 in the plasma specimens of thoracic aortic aneurysm patients in Example 2.
[Figure 4] Figure 4 includes graphs showing the ROC curves of TSP1, the combination of TSP1 and NPC2, the combination of TSP1 and IGFBP7, and the combination of TSP1, NPC2 and IGFBP7 in the diagnosis of thoracic aortic aneurysm in Example 2.
[Figure 5] Figure 5 is a graph showing the results of the measurement (ELISA method) of PROF1 in the serum specimens of thoracic aortic aneurysm patients in Example 3.
[Figure 6] Figure 6 includes graphs showing the ROC curves of PROF1, the combination of PROF1 and NPC2, the combination of PROF1 and IGFBP7, and the combination of PROF1, NPC2 and IGFBP7 in the diagnosis of thoracic aortic aneurysm in Example 3.
[Figure 7] Figure 7 includes graphs showing the results of the measurement (ELISA method) of NPC2 and IGFBP7 in the serum specimens of abdominal aortic aneurysm patients in Example 4.
[Figure 8] Figure 8 includes graphs showing the ROC curves of NPC2, IGFBP7, and the combination thereof in the diagnosis of abdominal aortic aneurysm in Example 4.
[Figure 9] Figure 9 is a graph showing the results of the measurement (ELISA method) of TSP1 in the plasma specimens of abdominal aortic aneurysm patients in Example 5.
[Figure 10] Figure 10 includes graphs showing the ROC curves of TSP1, the combination of TSP1 and NPC2, the combination of TSP1 and IGFBP7, and the combination of TSP1, NPC2 and IGFBP7 in the diagnosis of abdominal aortic aneurysm in Example 5.
[Figure 11] Figure 11 is a graph showing the results of the measurement (ELISA method) of PROF1 in the serum specimens of abdominal aortic aneurysm patients in Example 6.
[Figure 12] Figure 12 includes graphs showing the ROC curves of PROF1, the combination of PROF1 and NPC2, the combination of PROF1 and IGFBP7, and the combination of PROF1, NPC2 and IGFBP7 in the diagnosis of abdominal aortic aneurysm in Example 6.
[Figure 13] Figure 13 includes graphs showing the results of the measurement (latex agglutination method) of CRP in the serum specimens of thoracic aortic aneurysm patients in Comparative Example 1 and the ROC curve thereof.
[Figure 14] Figure 14 includes graphs showing the results of the measurement (latex agglutination method) of CRP in the serum specimens of abdominal aortic aneurysm patients in Comparative Example 2 and the ROC curve thereof.
[Figure 15] Figure 15 is a graph showing the results of the measurement (ELISA method) of IGFBP7 in the serum specimens of patients with other diseases (colorectal cancer, stomach cancer, non-small cell lung cancer, and hepatocellular carcinoma) and thoracic aortic aneurysm patients in Example 7.
[Figure 16] Figure 16 is a graph showing the results of the measurement (ELISA method) of IGFBP7 in the serum specimens of patients with other diseases (colorectal cancer, stomach cancer, non-small cell lung cancer, and hepatocellular carcinoma) and abdominal aortic aneurysm patients in Example 8.

### Description of Embodiments

For the purpose of identifying an aortic aneurysm marker, the present inventors have performed the following proteome analysis, using aortic media smooth muscle layer tissues collected from 14 cases of aortic tissues of healthy subjects and 29 cases of tissues of thoracic aortic aneurysm patients.

### - Tissue disruption and trypsin digestion

After collection of the tissues, the frozen tissues were disrupted using a bead disruptor, and thereafter, Lys-C (Lysyl Endopeptidase) and trypsin were added to the resulting tissues. The obtained mixture was incubated at 37°C overnight for digestion. The obtained digested peptide was desalinated using a C18 (octadecylsilyl) column to prepare an analysis sample.

### - Analysis

The analysis sample was separated using Nano LC (Hitachi Nano Frontier, Hitachi High-Technologies Corporation), and was then subjected to MS/MS analysis using connected Triple TOF 5600 (SCIEX). From the measurement data, proteins were identified using Mascot database search (Matrix science), and were then quantified using 2DICAL (2 Dimensional Image Converted Analysis of Liquid chromatography mass spectrometry, MITSUI KNOWLEDGE INDUSTRY CO., LTD.) that is comparative quantification proteome analysis software, in which LC-MS data were integrated and used, so that proteins having a difference between a normal aorta group and a thoracic aortic aneurysm group were searched. As a result, NPC2, IGFBP7, TSP1 and PROF1 were identified as proteins exhibiting a more significant increase in the thoracic aortic aneurysm group than in the normal aorta group, thereby completing the present invention.

As described above, it has been confirmed that the expression of all of NPC2, IGFBP7, TSP1 and PROF1 is augmented in the aortic media tissues derived from thoracic aortic aneurysm patients, in comparison to in the aortic media tissues of healthy subjects. By using NPC2 or IGFBP7 alone as a single marker, aortic aneurysm can be detected. However, as described in the Examples later, by combining NPC2 with IGFBP7 and using it as a marker, it exhibits more excellent detection effects. In addition, by combining NPC2 and/or IGFBP7 with TSP1 and/or PROF1, the detection effects can be further improved. As described in the Examples later, in the evaluation of markers of thoracic aortic aneurysm, both NPC2 and IGFBP7 have an AUC value of greater than 0.9 when they are used alone, and thus, NPC2 and IGFBP7 are extremely useful markers. However, the combination of NPC2 and IGFBP7, the combination of NPC2 and TSP1, the combination of NPC2 and PROF1, the combination of IGFBP7 and PROF1, the combination of NPC2, IGFBP7 and TSP1, and the combination of NPC2, IGFBP7 and PROF1, each exhibit a higher AUC value, and thus, these combined uses are further useful as markers of thoracic aortic aneurysm. When ROC curves were produced based on the data of Examples 1 to 3 and the AUC values were then obtained, the AUC value of the combination of NPC2, TSP1 and PROF1 was 0.953, and the AUC value of the combination of NPC2, IGFBP7, TSP1 and PROF1 was 0.948 (wherein the ROC curves of the combinations of these markers are not shown in the figures). These combinations are all extremely useful as markers of thoracic aortic aneurysm.

Moreover, in the evaluation of markers of abdominal aortic aneurysm, NPC2 is a useful marker having an AUC value of 0.827. However, the combination of NPC2 and TSP1, and the combination of NPC2 and PROF1 are useful markers having higher AUC values. Furthermore, IGFBP7 alone, the combination of NPC2 and IGFBP7, the combination of IGFBP7 and TSP1, the combination of IGFBP7 and PROF1, the combination of NPC2, IGFBP7 and TSP1, and the combination of NPC2, IGFBP7 and PROF1 each have an AUC value of greater than 0.9, and thus, these are extremely useful as markers of abdominal aortic aneurysm. When ROC curves were produced based on the data of Examples 4 to 6 and the AUC values were then obtained, the AUC value of the combination of TSP1 and PROF1 was 0.837, and the AUC value of the combination of NPC2, TSP1 and PROF1 was 0.886 (wherein the ROC curves of the combinations of these markers are not shown in the figures). These combinations are all useful as markers of abdominal aortic aneurysm. Furthermore, when ROC curves were produced based on the data of Examples 4 to 6 and the AUC values were then obtained, the AUC value of the combination of IGFBP7, TSP1 and PROF1 was 0.952, and the AUC value of the combination of NPC2, IGFBP7, TSP1 and PROF1 was 0.952 (wherein the ROC curves of the combinations of these markers are not shown in the figures). These combinations are all extremely useful as markers of abdominal aortic aneurysm.

As explained above, the present invention is characterized in that NPC2 and/or IGFBP7 are used as a detection marker(s) of aortic aneurysm.

In the present invention, the "marker" or "biomarker" means a molecule used as a measurement target of a sample obtained from a subject.

The detection marker of aortic aneurysm according to the present invention comprises NPC2 and/or IGFBP7. Human NPC2 (i.e., Niemann-Pick disease type C2 protein) is a secretory protein having UniProt Accession No. P61916 (http://www.uniprot.org/uniprot/P61916). The amino acid sequence of human NPC2 is as set forth in SEQ ID NO: 1. In the amino acid sequence as set forth in SEQ ID NO: 1, the amino acid sequence portion consisting of the amino acids at positions 1 to 19 is a signal peptide, and the amino acid sequence portion consisting of the amino acids at positions 20 to 151 is a mature protein. Human NPC2 is a glycoprotein consisting of 132 amino acids, and is present abundantly in the epididymidis. Human NPC2 is also present in many other tissues. It is considered that NPC2 has cholesterol-binding ability, and that NPC2, together with NPC1, is associated with cholesterol excretion from lysosome. The loss-of-function of NPC2 causes Niemann-Pick disease type C involving abnormal accumulation of cholesterol in lysosome (Marie T. Vanier, Gilles Millat. Structure and function of the NPC2 protein. Biochimica et Biophysica Acta 2004; 1685: 14-21).

In the present invention, the NPC2 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 20 to 151 in the amino acid sequence as set forth in SEQ ID NO: 1, and having cholesterol-binding activity and/or the activity of excreting cholesterol from lysosome, or not having the aforementioned activity, such as a loss-of-function mutant. The NPC2 protein further includes multimers (a dimer or more) of the aforementioned proteins.

On the other hand, human IGFBP7 (i.e., Insulin-like growth factor-binding protein 7) is a secretory protein having UniProt Accession No. Q16270 (http://www.uniprot.org/uniprot/Q16270). The amino acid sequence of human IGFBP7 is as set forth in SEQ ID NO: 2. In the amino acid sequence as set forth in SEQ ID NO: 2, the amino acid sequence portion consisting of the amino acids at positions 1 to 26 is a signal peptide, and the amino acid sequence portion consisting of the amino acids at positions 27 to 282 is a mature protein. IGFBP7 is expressed in many tissues such as heart, brain, placenta, lung, liver, skeletal muscle, kidney, pancreas, spleen, thymus, prostate, testis, ovary, small intestine, and colon. It has been known that, differing from other IGFBP proteins, the IGFBP7 protein has weak binding ability to an insulin-like growth factor (IGF) and strongly binds to insulin. It has been reported that IGFBP7 is associated with regulation of cell growth, apoptosis, cellular aging, and angiogenesis (Shuzhen Zhu, Fangying Xu, Jing Zhang, Wenjing Ruan, Maode Lai. Insulin-like growth factor binding protein-related protein 1 and cancer. Clinica Chimica Acta 2014; 431: 23-32).

In the present invention, the IGFBP7 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 27 to 282 in the amino acid sequence as set forth in SEQ ID NO: 2, and having insulin-binding activity or not having the activity. The IGFBP7 protein further includes multimers (a dimer or more) of the aforementioned protein.

The present invention may include TSP1 as a marker that is used in combination with NPC2 and/or IGFBP7. Human TSP1 (i.e., Thrombospondin 1) is a secretory protein having UniProt Accession No. P07996 (http://www.uniprot.org/uniprot/P07996). The amino acid sequence of human TSP1 is as set forth in SEQ ID NO: 3. In the amino acid sequence as set forth in SEQ ID NO: 3, the amino acid sequence portion consisting of the amino acids at positions 1 to 18 is a signal peptide, and the amino acid sequence portion consisting of the amino acids at positions 19 to 1170 is a mature protein. TSP1 is a 450-kDa glycoprotein that forms a homotrimer, and is expressed in platelets, vascular smooth muscle cells, vascular endothelial cells, fibroblasts, and the like. It has been known that TSP1 is associated with vascular structure and the maintenance of homeostasis, and further, it has been reported that TSP1 binds to many receptors, proteases, growth factors, etc. (Smriti Murali Krishna, Jonathan Golledge. The role of thrombospondin-1 in cardiovascular health and pathology. International Journal of Cardiology 2013; 168: 692-706).

In the present invention, the TSP1 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 19 to 1170 in the amino acid sequence as set forth in SEQ ID NO: 3, and further includes multimers (a dimer or more) of the aforementioned protein.

The present invention may include PROF1 as a marker that is used in combination with NPC2 and/or IGFBP7. Human PROF1 (i.e., Profilin 1) is a protein having UniProt Accession No. P07737 (https://www.uniprot.org/uniprot/P07737). The amino acid sequence of human PROF1 is as set forth in SEQ ID NO: 4. PROF1 is a 15-kDa actin-binding protein composed of ubiquitously expressed and highly conserved 140 amino acid residues, and PROF1 has the function of catalyzing polymerization of actin in a concentration-dependent manner (Duah Alkam, Ezra Z. Feldman, Awantika Singh, and Mahmoud Kiaei. Profilin1 Biology and its Mutation, Actin(g) in Disease. Cell Mol Life Sci. 2017; 74(6): 967-981).

In the present invention, the PROF1 protein includes a protein consisting of an amino acid sequence having sequence identity of 70% or more, 75% or more, 80% or more, 85% or more, or 90% or more, more preferably 95% or more, 96% or more, 97% or more, 98% or more, or 99% or more, and most preferably 100%, to the amino acid sequence portion consisting of the amino acids at positions 1 to 140 in the amino acid sequence as set forth in SEQ ID NO: 4, and having actin-binding activity. The PROF1 protein further includes multimers (a dimer or more) of the aforementioned protein.

Besides, the marker according to the present invention can also be used in combination with known aortic aneurysm markers, such as C-reactive protein (CRP), D-dimer, white blood cell count (WBC), plasma homocysteine, or matrix metalloproteinase-9 (MMP-9).

The onset site of aortic aneurysm that can be detected using the marker according to the present invention is not particularly limited. All of thoracic aortic aneurysm, thoracoabdominal aortic aneurysm, and abdominal aortic aneurysm, which develop in any of aortic root, ascending aorta, arch of aorta, and descending aorta, can be detected using the present marker.

In the present invention, the method for detecting aortic aneurysm comprises a step of measuring an NPC2 protein and/or an IGFBP7 protein. Upon the measurement of an NPC2 protein and/or an IGFBP7 protein in a sample, NPC2 and/or IGFBP7 serving as a glycoprotein(s) may be measured, or after the removal of a post-translational modification such as a sugar chain or phosphoric acid by a pre-treatment, the amino acid sequence portion(s) of NPC2 and/or IGFBP7 may be measured.

Moreover, in the present invention, the method for detecting aortic aneurysm may further comprise a step of measuring TSP1 and/or PROF1, in addition to the step of measuring an NPC2 protein and/or an IGFBP7 protein. Upon the measurement of a TSP1 protein and/or a PROF1 protein in a sample, TSP1 and/or PROF1 serving as a glycoprotein(s) may be measured, or after the removal of a post-translational modification such as a sugar chain or phosphoric acid by a pre-treatment, the amino acid sequence portion(s) of TSP1 and/or PROF1 may be measured.

In the method for detecting aortic aneurysm, the marker according to the present invention is measured in a sample obtained from a subject, so that the aortic aneurysm of the subject can be detected. Accordingly, the method for detecting aortic aneurysm can be said to be a method of diagnosing, testing, evaluating or determining aortic aneurysm, or a method of collecting *in vitro* data for detecting aortic aneurysm, or the like.

Examples of the animal that can be a test subject used in the method for detecting aortic aneurysm may include mammals such as a human, a monkey, a bovine, a swine, a horse, a dog, a cat, sheep, a goat, a rabbit, a hamster, a Guinea pig, a mouse and a rat, and the animal used herein is preferably a human.

In addition, examples of the sample used in the method for detecting aortic aneurysm may include serum, plasma, and blood.

In the present invention, for example, the NPC2 protein level and/or the IGFBP7 protein level are measured *in vitro* in a sample collected from a patient suspected of suffering from aortic aneurysm, so that the possibility of onset of aortic aneurysm can be determined. When the NPC2 protein level and/or IGFBP7 protein level in a patient specimen is compared with the level(s) in a healthy subject and as a result, the NPC2 protein level and/or IGFBP7 protein level in the patient specimen are higher than the level(s) in the healthy subject, it can be determined that it is highly likely that aortic aneurysm will develop in future or the aortic aneurysm currently progresses in any site of the aorta.

In the present invention, by measuring the TSP1 protein level and/or the PROF1 protein level, as well as the measurement of the NPC2 protein level and/or the IGFBP7 protein level, the possibility of onset of aortic aneurysm can also be determined. When the TSP1 protein level and/or the PROF1 protein level in a patient specimen, as well as the NPC2 protein level and/or the IGFBP7 protein level in the patient specimen, is compared with the level(s) in a healthy subject, and as a result, the NPC2 protein level and/or the IGFBP7 protein level in the patient specimen are higher than the level(s) in the healthy subject, and further, the TSP1 protein level and/or the PROF1 protein level in the patient specimen are lower than the level(s) in the healthy subject, it can be determined that it is highly likely that aortic aneurysm will develop in future or the aortic aneurysm currently progresses in any site of the aorta.

Specifically, in accordance with the method for detecting aortic aneurysm, the present invention further includes a method of detecting or screening a subject having a risk of developing aortic aneurysm in future by measuring the marker according to the present invention in a sample obtained from the subject.

Moreover, the cutoff value of each marker for determining that it is highly likely that aortic aneurysm will develop in future or aortic aneurysm currently progresses is not limited, but examples of the cutoff value are the following. The following cutoff values are each obtained by calculating the accuracy of each cutoff based on the ROC curve produced from the data shown in the Examples and then selecting the cutoff value exhibiting the highest accuracy.

(1) Thoracic aortic aneurysm: cutoff values in serum:
   (i) Cutoff value of NPC2: 4.2 ng/mL (sensitivity: 96.6%; specificity: 88.6%; and accuracy: 91.8%);
   (ii) Cutoff values of IGFBP7 (in the case of thoracic aortic aneurysm, three cutoff values having the highest accuracy (84.9%) were obtained):
      Cutoff value 1: 175.4 ng/mL (sensitivity: 75.9%; specificity: 90.9%; and accuracy: 84.9%);
      Cutoff value 2: 173.5 ng/mL (sensitivity: 79.3%; specificity: 88.6%; and accuracy: 84.9%);
      and
      Cutoff value 3: 193.9 - 209.2 ng/mL (sensitivity: 62.1%; specificity: 100%; and accuracy: 84.9%); and
   (iii) Cutoff value of PROF1: 2.1 ng/mL (sensitivity: 58.6%; specificity: 88.6%; and accuracy: 76.7%).
(2) Thoracic aortic aneurysm: cutoff value in plasma:
   Cutoff value of TSP1: 874.0 ng/mL (sensitivity: 62.1%; specificity: 37.9%; and accuracy: 78.1%).
(3) Abdominal aortic aneurysm: cutoff values in serum:
   (i) Cutoff value of NPC2: 3.9 ng/mL (sensitivity: 80.4%; specificity: 86.4%; and accuracy: 83.2%);
   (ii) Cutoff values of IGFBP7 (in the case of abdominal aortic aneurysm, two cutoff values having the highest accuracy (84.2%) were obtained):
      Cutoff value 1: 175.4 ng/mL (sensitivity: 78.4%; specificity: 90.9%; and accuracy: 84.2%);
      Cutoff value 2: 173.5 ng/mL (sensitivity: 80.4%; specificity: 88.6%; and accuracy: 84.2%);
      and
   (iii) Cutoff value of PROF1 (the number of abdominal aortic aneurysm cases measured was 41, which was different from the number of abdominal aortic aneurysm cases measured using other markers, and two cutoff values having the highest accuracy (64.2%) were obtained):
      Cutoff value 1: 2.9 ng/mL (sensitivity: 75.6%; specificity: 68.2%; and accuracy: 64.2%); and
      Cutoff value 2: 3.1 ng/mL (sensitivity: 82.9%; specificity: 61.4%; and accuracy: 64.2%).
(4) Abdominal aortic aneurysm: cutoff value in plasma:
   Cutoff value of TSP1: 1128.0 ng/mL (sensitivity: 74.5%; specificity: 75.0%; and accuracy: 74.7%).

On the basis of these cutoff values, when the NPC2 protein level and/or the IGFBP7 protein level in a patient specimen are higher than the cutoff values, and further, when the TSP1 protein level and/or the PROF1 protein level in the patient specimen are lower than the cutoff values, it can be determined that it is highly likely that thoracic aortic aneurysm or abdominal aortic aneurysm will be developed, or that the thoracic aortic aneurysm or the abdominal aortic aneurysm currently progresses.

The marker according to the present invention can also be used in the screening of a preventive or therapeutic agent for aortic aneurysm, or the evaluation or determination of the effects of a preventive or therapeutic agent for aortic aneurysm. For example, when the effects of a preventive or therapeutic agent for aortic aneurysm are evaluated or determined, samples are collected from a test subject animal in need of prevention or treatment of aortic aneurysm, before and after the preventive or therapeutic agent for aortic aneurysm is administered to the test subject animal, or samples are collected from the test subject animal in two or more time points in chronological order, after administration of the preventive or therapeutic agent, and thereafter, a change over time in the NPC2 protein levels and/or the IGFBP7 protein levels in the samples is examined, so that the effects of the preventive or therapeutic agent for aortic aneurysm can be evaluated or determined. When the formation or progression of an aortic aneurysm lesion is suppressed by administration of a preventive or therapeutic agent for aortic aneurysm, an increase over time in the NPC2 protein level and/or the IGFBP7 protein level in the sample collected from the test subject animal tends to be suppressed, or the level(s) remain unchanged. On the other hand, when the aortic aneurysm lesion is ameliorated by administration of the preventive or therapeutic agent for aortic aneurysm, the NPC2 protein level and/or the IGFBP7 protein level in the sample collected from the test subject animal tends to be decreased over time.

In the present invention, samples are collected from a test subject animal in need of prevention or treatment of aortic aneurysm, before and after the preventive or therapeutic agent for aortic aneurysm is administered to the test subject animal. Otherwise, samples are collected from the test subject animal in two or more timepoints in chronological order, after administration of the preventive or therapeutic agent, and a change over time in the NPC2 protein levels and/or the IGFBP7 protein levels in the samples is examined, and further, a change over time in the TSP1 protein levels and/or the PROF1 proteins levels in the samples is also examined, so that the effects of the preventive or therapeutic agent for aortic aneurysm can be evaluated or determined. When the formation or progression of an aortic aneurysm lesion is suppressed by administration of a preventive or therapeutic agent for aortic aneurysm, an increase over time in the NPC2 protein level and/or the IGFBP7 protein level in the sample collected from the test subject animal tends to be suppressed, or the level(s) remain unchanged, and also a decrease over time in the TSP1 protein level and/or the PROF1 protein level in the sample collected from the test subject animal tends to be suppressed, or the level(s) remain unchanged. On the other hand, when the aortic aneurysm lesion is ameliorated by administration of the preventive or therapeutic agent for aortic aneurysm, the NPC2 protein level and/or the IGFBP7 protein level in the sample collected from the test subject animal tends to be decreased over time, and further, the TSP1 protein level and/or the PROF1 protein level in the sample collected from the test subject animal tends to be increased over time.

As methods of measuring the marker protein according to the present invention, all of publicly known methods may be applied, as long as the methods are capable of specifically measuring an NPC2 protein, an IGFBP7 protein, a TSP1 protein and a PROF1 protein. Examples of the methods may include immunoassay and mass spectrometry. As a detection agent used upon the measurement of the marker protein according to the present invention, an antibody, an aptamer, or the like can be used.

The immunoassay used herein is not particularly limited. Examples of the immunoassay may include various types of enzyme immunoassay, radioimmunoassay (RIA), enzyme-linked immunosorbent assay (ELISA), double monoclonal antibody sandwich assay, monoclonal-polyclonal antibody sandwich assay, an immunostaining method, an immunofluorescence method, a Western blotting method, a biotin-avidin method, an immunoprecipitation method, a gold colloidal aggregation method, an immunochromatography method, latex agglutination method (LA), and turbidimetric immunoassay (TIA).

As a detection agent used in the immunoassay, an anti-NPC2 antibody, an anti-IGFBP7 antibody, an anti-TSPl antibody, and an anti-PROF1 antibody, which have already been commercially available, may be used. Otherwise, such antibodies may be prepared according to a common method, on the basis of the amino acid sequence (SEQ ID NO: 1) of NPC2, the amino acid sequence (SEQ ID NO: 2) of IGFBP7, the amino acid sequence (SEQ ID NO: 3) of TSP1, and the amino acid sequence (SEQ ID NO: 4) of PROF1, all of which have been known. The antibody may be an antibody specifically recognizing the structure of the amino acid sequence of each of NPC2, IGFBP7, TSP1, and PROF1, or may also be an antibody specifically recognizing the entire structure including post-translational modification such as each sugar chain, disulfide bond, phosphorylation, etc.

The animal species or clone, from which the antibody is derived, is not particularly limited, as long as the antibody is capable of detecting the NPC2 protein, the IGFBP7 protein, the TSP1 protein, or the PROF1 protein. Examples of the antibody may include antibodies derived from a rabbit, a goat, a mouse, a rat, a Guinea pig, a horse, sheep, a camel, a chicken and the like. The antibody may be either a monoclonal antibody or a polyclonal antibody. In addition, antibodies of all subclasses suitable for specific binding to the NPC2 protein, the IGFBP7 protein, the TSP1 protein, and the PROF1 protein can be used. Recombinant antibodies or fragments such as a Fab, Fab' or F(ab')2 fragment can be naturally used.

As an agent for detecting aortic aneurysm according to the present invention, an aptamer having binding ability to the NPC2 protein, the IGFBP7 protein, the TSP1 protein, or the PROF1 protein can also be utilized. For the production of such an aptamer, a nucleic acid aptamer that is bindable DNA or RNA or a derivative thereof, or a peptide aptamer composed of amino acids, may be synthesized from the amino acid sequence (SEQ ID NO: 1) of NPC2, the amino acid sequence (SEQ ID NO: 2) of IGFBP7, the TSP1 protein (SEQ ID NO: 3), or the PROF1 protein(SEQ ID NO: 4), each of which has been known, according to a publicly known method, and may be then used. Upon the measurement, the binding of such an aptamer can be detected by a luminescence method, a fluorescence method, or a surface plasmon resonance method.

The mass spectrometry used herein is not particularly limited. A mass spectrometer, in which the ion source of electrospray ionization (ESI), matrix assisted laser desorption ionization (MALDI), surface enhanced laser desorption ionization (SELDI), etc. is combined with time-of-flight mass spectrometer (TOF), ion trap mass spectrometer (IT), Fourier-transform mass spectrometer (FT), etc., can be utilized. LC-MS, CE-MS, etc., in which a mass spectrometer is connected with a separation device such as high performance liquid chromatography (HPLC) or capillary electrophoresis (CE), can be used. Moreover, examples of a method of obtaining mass spectrometry data may include data-independent acquisition (DIA), data-dependent acquisition (DDA), and multiple reaction monitoring (MRM). The mass spectrometry also includes the case of performing stable isotope labeling on a sample, using iTRAQ reagent (SCIEX), etc.

Various types of reagents necessary for performing the detection of aortic aneurysm of the present invention may have previously been packaged and prepared as a kit. For example, necessary reagents, such as (i) a monoclonal antibody or polyclonal antibody that is used as a capture antibody specific to the marker protein according to the present invention, (ii) an enzyme-labeled monoclonal antibody or polyclonal antibody that is used as a detection antibody specific to the marker protein according to the present invention, and (iii) a substrate solution, are provided as a kit.

### Examples

Hereinafter, the present invention will be described in more detail in the following examples. However, the following examples are not intended to limit the technical scope of the present invention.

### [Example 1]

### Measurement of NPC2 and IGFBP7 in serum specimens of thoracic aortic aneurysm patients (ELISA method)

The serum concentrations of NPC2 and IGFBP7 were measured according to ELISA. NPC2 and IGFBP7 were measured using NPC2 ELISA Kit (Aviva Systems Biology) and ELISA Kit for Insulin Like Growth Factor Binding Protein 7 (Cloud-Clone), respectively. The measurement was carried out in accordance with the protocols included with the kits.

Specifically, a diluted serum specimen was added to a plate on which an anti-NPC2 antibody or an anti-IGFBP7 antibody had been immobilized, and was then incubated. Thereafter, the specimen solution was removed. In the case of the measurement of NPC2, a biotin-labeled anti-NPC2 antibody was added, and in the case of the measurement of IGFBP7, a detection reagent A solution was added, followed by incubation. After completion of the incubation, a washing operation was performed. In the case of the measurement of NPC2, HRP (Horseradish peroxidase)-labeled avidin was added, and in the case of the measurement of IGFBP7, a detection reagent B solution was added, followed by incubation. Subsequently, a washing operation was performed, a TMB (3,3',5,5'-tetramethylbenzidine) solution was then added, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the concentration of NPC2 and the concentration of IGFBP7 were measured. StatFlex Ver. 6.0 (Artec Co., Ltd.) was used for the statistical treatment, and Mann-Whitney U Test was used for the significance test.

### - Result 1

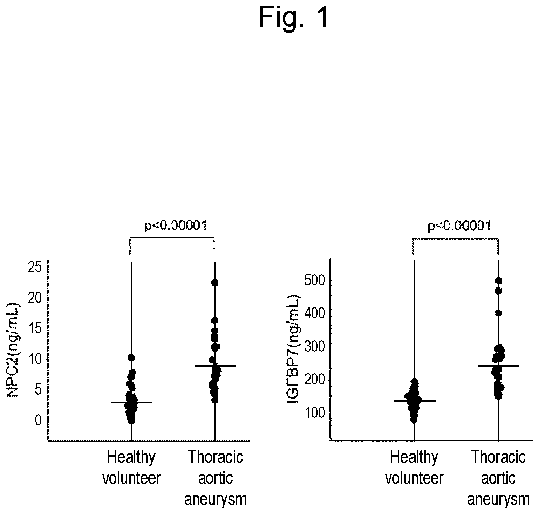

The results of NPC2 and IGFBP7 are shown in Figure 1.

From the results shown in Figure 1, the serum NPC2 values were 2.941±2.001 ng/mL in healthy subject (44 cases) and 9.021±4.313 ng/mL in thoracic aortic aneurysm patients (29 cases), respectively. Thus, the serum NPC2 value was significantly increased in the thoracic aortic aneurysm patients. Therefore, NPC2 was demonstrated to be a useful thoracic aortic aneurysm marker.

On the other hand, the serum IGFBP7 values were 139.216±28.481 ng/mL in healthy subject (44 cases) and 243.941±89.474 ng/mL in thoracic aortic aneurysm patients (29 cases), respectively. Thus, the serum IGFBP7 value was significantly increased in the thoracic aortic aneurysm patients. Therefore, IGFBP7 was also demonstrated to be a useful thoracic aortic aneurysm marker.

### - Result 2 (Evaluation of NPC2 and IGFBP7 as thoracic aortic aneurysm markers based on ROC curves)

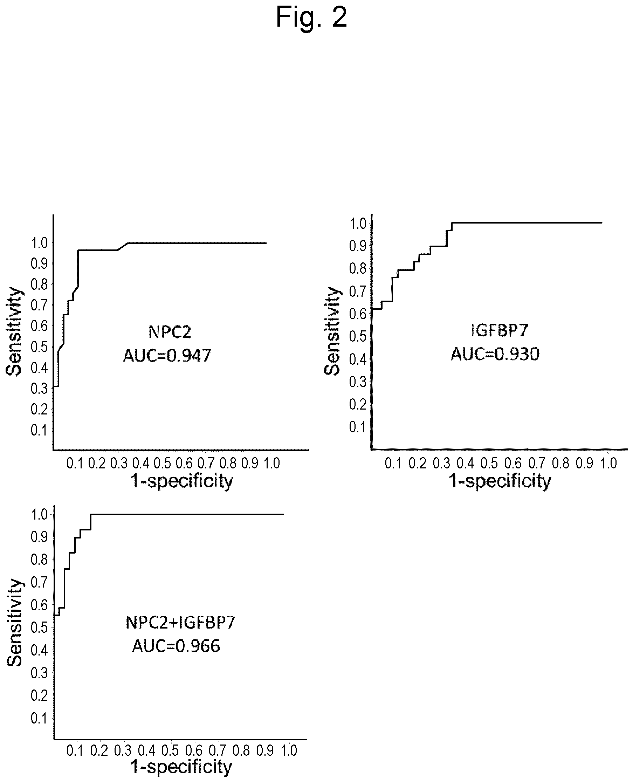

The ROC (Receiver Operating Characteristic) curves of NPC2 and IGFBP7 in the diagnosis of thoracic aortic aneurysm are shown in Figure 2. The ROC curves were produced using statistical analysis software StatFlex Ver. 6.0 (Artec Co., Ltd.). The AUC (Area Under the Curve) value obtained from the ROC curve was 0.947 in the case of NPC2, and was 0.930 in the case of IGFBP7. In addition, the AUC value obtained from the combination of NPC2 and IGFBP7 was 0.966. Therefore, it was demonstrated that both NPC2 and IGFBP7 are useful thoracic aortic aneurysm markers, and that the usefulness is further increased by combining NPC2 with IGFBP7.

### [Example 2]

### Measurement of TSP1 in plasma specimens of thoracic aortic aneurysm patients (ELISA method), and evaluation of TSP1 as thoracic aortic aneurysm marker based on ROC curves

### (combination of TSP1 with NPC2 and IGFBP7)

The plasma concentration of TSP1 was measured according to ELISA. TSP1 was measured using Human Thrombospondin-1 Quantikine ELISA Kit (R&D Systems). The measurement was carried out in accordance with the protocols included with the kit.

Specifically, a diluted plasma specimen was added to a plate on which an anti-TSPl antibody had been immobilized, followed by incubation. Thereafter, the specimen solution was removed, and a peroxidase-binding anti-TSPl antibody was added, followed by incubation. After completion of the incubation, a washing operation was performed, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the TSP1 concentration was measured. StatFlex Ver. 6.0 (Artec Co., Ltd.) was used for the statistical treatment, and Mann-Whitney U Test was used for the significance test.

### - Result 1

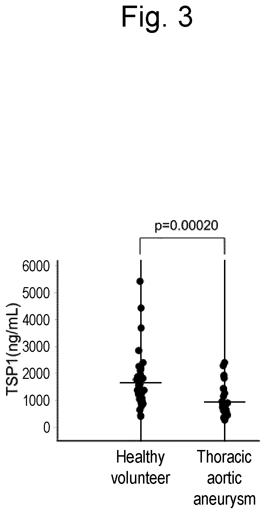

The results of TSP1 are shown in Figure 3.

From the results shown in Figure 3, the plasma TSP1 values were 1666.55±968.196 ng/mL in healthy subject (44 cases) and 950.152±611.083 ng/mL in thoracic aortic aneurysm patients (29 cases), respectively. Thus, the plasma TSP1 value was significantly decreased in the thoracic aortic aneurysm patients. Therefore, TSP1 was demonstrated to be a useful thoracic aortic aneurysm marker.

### - Result 2 (Evaluation of TSP1 as thoracic aortic aneurysm marker based on ROC curves)

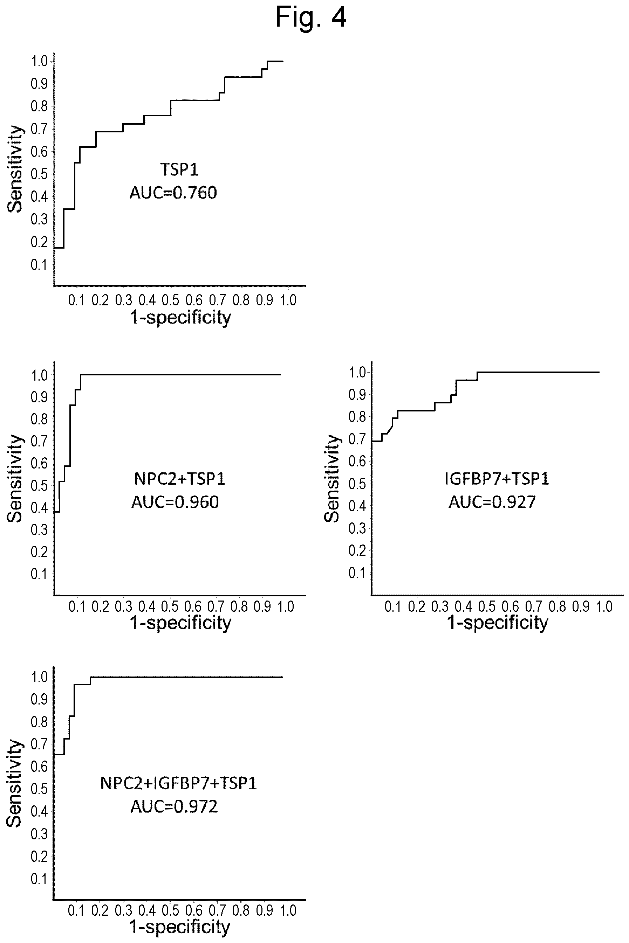

The ROC (Receiver Operating Characteristic) curves of TSP1 in the diagnosis of thoracic aortic aneurysm are shown in Figure 4. The ROC curves were produced using statistical analysis software StatFlex Ver. 6.0 (Artec Co., Ltd.). The AUC (Area Under the Curve) value of TSP1 obtained from the ROC curve was 0.760. In addition, the AUC values were obtained from the ROC curve regarding the combinations of TSP1 with NPC2 and IGFBP7, which were measured by the method described in Example 1. As a result, the AUC value was 0.960 when TSP1 was combined with NPC2, and the AUC value was 0.927 when TSP1 was combined with IGFBP7. Also, the AUC value was 0.972 in the combination of TSP1, NPC2 and IGFBP7. Therefore, it was demonstrated that TSP1 is a useful thoracic aortic aneurysm marker, and that the usefulness is further increased by combining TSP1 with NPC2 and/or IGFBP7.

### [Example 3]

### Measurement of PROF1 in serum specimens of thoracic aortic aneurysm patients (ELISA method), and evaluation of PROF1 as thoracic aortic aneurysm marker based on ROC curves (combination of PROF1 with NPC2 and IGFBP7)

The serum concentration of PROF1 was measured according to ELISA. PROF1 was measured using Human Profilin 1 ELISA Kit (CUSABIO technology). The measurement was carried out in accordance with the protocols included with the kit.

Specifically, a diluted serum specimen was added to a plate on which an anti-PROF1 antibody had been immobilized, followed by incubation. Thereafter, the specimen solution was removed, and a biotin-labeled anti-PROF1 antibody was added, followed by incubation. After completion of the incubation, a washing operation was performed, and HRP (Horseradish peroxidase)-labeled avidin was added to the resultant, followed by incubation. Subsequently, a washing operation was performed, a TMB (3,3',5,5'-tetramethylbenzidine) solution was then added to the resultant, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the PROF1 concentration was measured. StatFlex Ver. 6.0 (Artec Co., Ltd.) was used for the statistical treatment, and Mann-Whitney U Test was used for the significance test.

### - Result 1

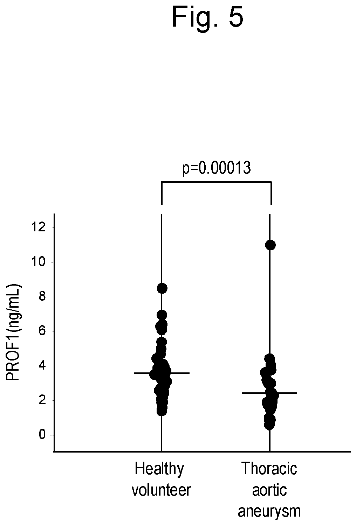

The results of PROF1 are shown in Figure 5.

From the results shown in Figure 5, the serum PROF1 values were 3.588±1.508 ng/mL in healthy subject (44 cases) and 2.456±1.943 ng/mL in thoracic aortic aneurysm patients (29 cases), respectively. Thus, the serum PROF1 value was significantly decreased in the thoracic aortic aneurysm patients. Therefore, PROF1 was demonstrated to be a useful thoracic aortic aneurysm marker.

### - Result 2 (Evaluation of PROF1 as thoracic aortic aneurysm marker based on ROC curves)

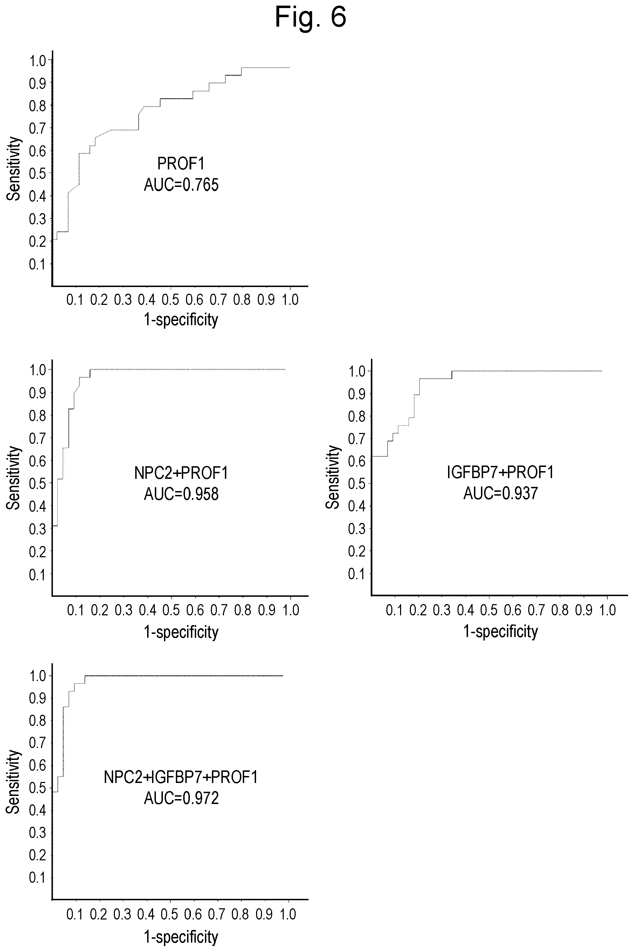

The ROC (Receiver Operating Characteristic) curves of PROF1 in the diagnosis of thoracic aortic aneurysm are shown in Figure 6. The ROC curves were produced using statistical analysis software StatFlex Ver. 6.0 (Artec Co., Ltd.). The AUC (Area Under the Curve) value of PROF1 obtained from the ROC curve was 0.765. In addition, the AUC values were obtained from the ROC curve regarding the combinations of PROF1 with NPC2 and IGFBP7, which were measured by the method described in Example 1. As a result, the AUC value was 0.958 when PROF1 was combined with NPC2, and the AUC value was 0.937 when PROF1 was combined with IGFBP7. Also, the AUC value was 0.972 in the combination of PROF1, NPC2 and IGFBP7. Therefore, it was demonstrated that PROF1 is a useful thoracic aortic aneurysm marker, and that the usefulness is further increased by combining PROF1 with NPC2 and/or IGFBP7.

### [Example 4]

### Measurement of NPC2 and IGFBP7 in serum specimens of abdominal aortic aneurysm patients (ELISA method)

The serum concentrations of NPC2 and IGFBP7 were measured according to ELISA. NPC2 and IGFBP7 were measured using NPC2 ELISA Kit (Aviva Systems Biology) and ELISA Kit for Insulin Like Growth Factor Binding Protein 7 (Cloud-Clone), respectively. The measurement was carried out in accordance with the protocols included with the kits.

Specifically, a diluted serum specimen was added to a plate on which an anti-NPC2 antibody or an anti-IGFBP7 antibody had been immobilized, and was then incubated. Thereafter, the specimen solution was removed. In the case of the measurement of NPC2, a biotin-labeled anti-NPC2 antibody was added, and in the case of the measurement of IGFBP7, a detection reagent A solution was added, followed by incubation. After completion of the incubation, a washing operation was performed. In the case of the measurement of NPC2, HRP (Horseradish peroxidase)-labeled avidin was added, and in the case of the measurement of IGFBP7, a detection reagent B solution was added, followed by incubation. Subsequently, a washing operation was performed, a TMB (3,3',5,5'-tetramethylbenzidine) solution was then added, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the concentration of NPC2 and the concentration of IGFBP7 were measured. StatFlex Ver. 6.0 (Artec Co., Ltd.) was used for the statistical treatment, and Mann-Whitney U Test was used for the significance test.

### - Result 1

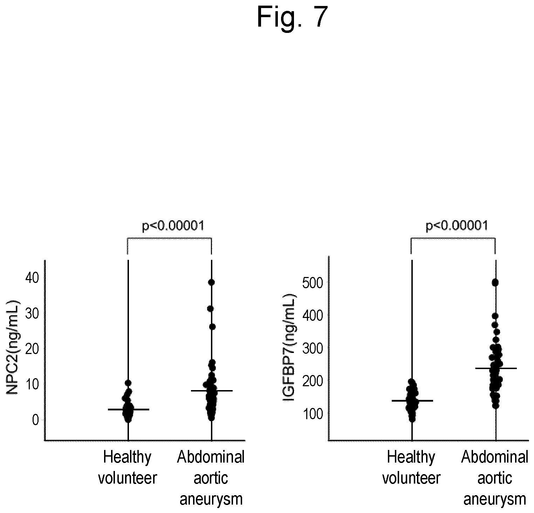

The results of NPC2 and IGFBP7 are shown in Figure 7.

From the results shown in Figure 7, the serum NPC2 values were 2.941±2.001 ng/mL in healthy subject (44 cases) and 8.125±7.088 ng/mL in abdominal aortic aneurysm patients (51 cases), respectively. Thus, the serum NPC2 value was significantly increased in the abdominal aortic aneurysm patients. Therefore, NPC2 was demonstrated to be a useful abdominal aortic aneurysm marker.

On the other hand, the serum IGFBP7 values were 139.216±28.481 ng/mL in healthy subject (44 cases) and 236.588±82.702 ng/mL in abdominal aortic aneurysm patients (51 cases), respectively. Thus, the serum IGFBP7 value was significantly increased in the abdominal aortic aneurysm patients. Therefore, IGFBP7 was also demonstrated to be a useful abdominal aortic aneurysm marker.

### - Result 2 (Evaluation of NPC2 and IGFBP7 as abdominal aortic aneurysm markers based on ROC curves)

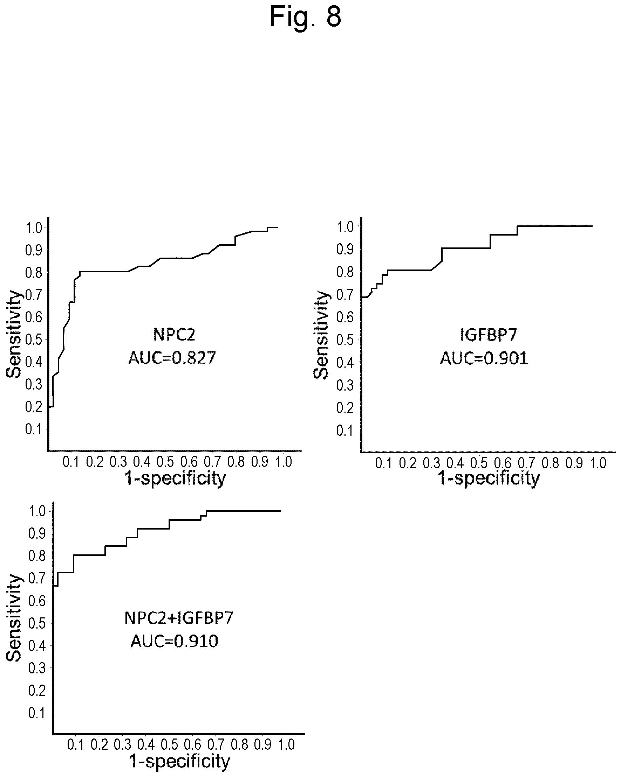

The ROC (Receiver Operating Characteristic) curves of NPC2 and IGFBP7 in the diagnosis of abdominal aortic aneurysm are shown in Figure 8. The ROC curves were produced using statistical analysis software StatFlex Ver. 6.0 (Artec Co., Ltd.). The AUC (Area Under the Curve) value obtained from the ROC curve was 0.827 in the case of NPC2, and was 0.901 in the case of IGFBP7. In addition, the AUC value obtained from the combination of NPC2 and IGFBP7 was 0.910. Therefore, it was demonstrated that both NPC2 and IGFBP7 are useful abdominal aortic aneurysm markers, and that the usefulness is further increased by combining NPC2 with IGFBP7.

### [Example 5]

### Measurement of TSP1 in plasma specimens of abdominal aortic aneurysm patients (ELISA method), and evaluation of TSP1 as abdominal aortic aneurysm marker based on ROC curves (combination of TSP1 with NPC2 and IGFBP7)

The plasma concentration of TSP1 was measured according to ELISA. TSP1 was measured using Human Thrombospondin-1 Quantikine ELISA Kit (R&D Systems). The measurement was carried out in accordance with the protocols included with the kit.

Specifically, a diluted plasma specimen was added to a plate on which an anti-TSPl antibody had been immobilized, followed by incubation. Thereafter, the specimen solution was removed, and a peroxidase-binding anti-TSPl antibody was added, followed by incubation. After completion of the incubation, a washing operation was performed, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the TSP1 concentration was measured. StatFlex Ver. 6.0 (Artec Co., Ltd.) was used for the statistical treatment, and Mann-Whitney U Test was used for the significance test.

### - Result 1

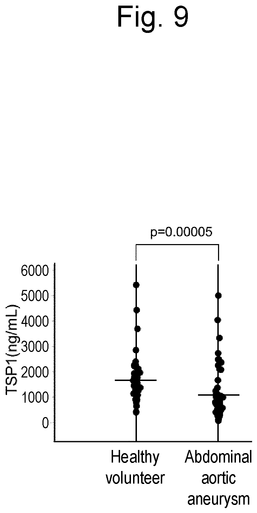

The results of TSP1 are shown in Figure 9.

From the results shown in Figure 9, the plasma TSP1 values were 1666.55±968.196 ng/mL in healthy subject (44 cases) and 1086.48±1041.75 ng/mL in abdominal aortic aneurysm patients (51 cases), respectively. Thus, the plasma TSP1 value was significantly decreased in the abdominal aortic aneurysm patients. Therefore, TSP1 was demonstrated to be a useful abdominal aortic aneurysm marker.

### - Result 2 (Evaluation of TSP1 as abdominal aortic aneurysm marker based on ROC curves)

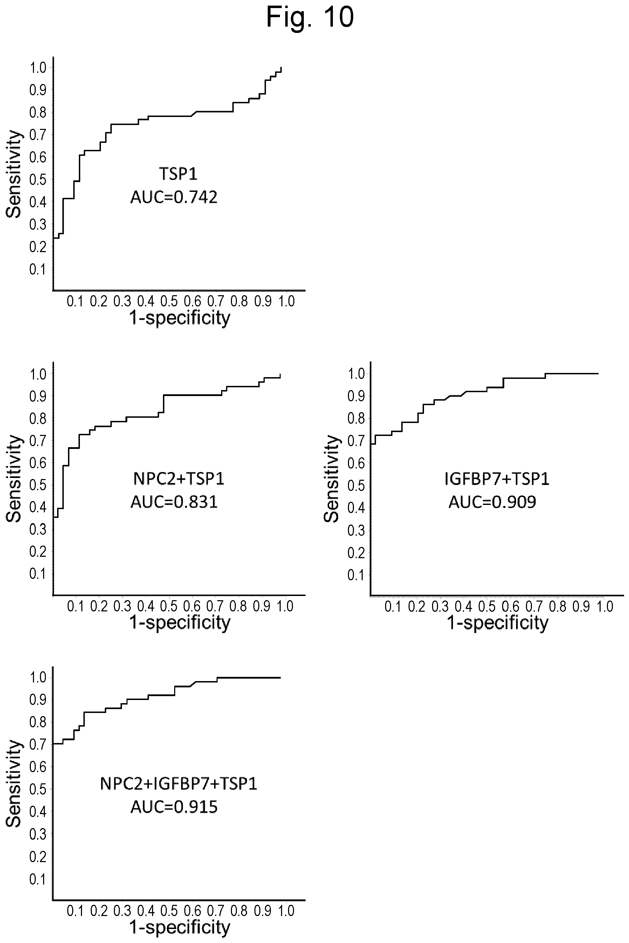

The ROC (Receiver Operating Characteristic) curves of TSP1 in the diagnosis of abdominal aortic aneurysm are shown in Figure 10. The ROC curves were produced using statistical analysis software StatFlex Ver. 6.0 (Artec Co., Ltd.). The AUC (Area Under the Curve) value of TSP1 obtained from the ROC curve was 0.742. In addition, the AUC values were obtained from the ROC curve regarding the combinations of TSP1 with NPC2 and IGFBP7, which were measured by the method described in Example 4. As a result, the AUC value was 0.831 when TSP1 was combined with NPC2, and the AUC value was 0.909 when TSP1 was combined with IGFBP7. Also, the AUC value was 0.915 in the combination of TSP1, NPC2 and IGFBP7. Therefore, it was demonstrated that TSP1 is a useful abdominal aortic aneurysm marker, and that the usefulness is further increased by combining TSP1 with NPC2 and/or IGFBP7.

### [Example 6]

### Measurement of PROF1 in serum specimens of abdominal aortic aneurysm patients (ELISA method), and evaluation of PROF1 as abdominal aortic aneurysm marker based on ROC curves (combination of PROF1 with NPC2 and IGFBP7)

The serum concentration of PROF1 was measured according to ELISA. PROF1 was measured using Human Profilin 1 ELISA Kit (CUSABIO technology). The measurement was carried out in accordance with the protocols included with the kit.

Specifically, a diluted serum specimen was added to a plate on which an anti-PROF1 antibody had been immobilized, followed by incubation. Thereafter, the specimen solution was removed, and a biotin-labeled anti-PROF1 antibody was added, followed by incubation. After completion of the incubation, a washing operation was performed, and HRP (Horseradish peroxidase)-labeled avidin was added to the resultant, followed by incubation. Subsequently, a washing operation was performed, a TMB (3,3',5,5'-tetramethylbenzidine) solution was then added to the resultant, and a coloration reaction was then carried out. Thereafter, a stop solution was added to the reaction mixture, and then, using a microplate reader (SpectraMax M2e, Molecular Devices), the measurement was carried out at a main wavelength of 450 nm and at a sub-wavelength of 540 nm. The concentration was calculated from the obtained data, based on a calibration curve produced from the measurement data of simultaneously measured each standard solution, and the PROF1 concentration was measured. StatFlex Ver. 6.0 (Artec Co., Ltd.) was used for the statistical treatment, and Mann-Whitney U Test was used for the significance test.

### - Result 1

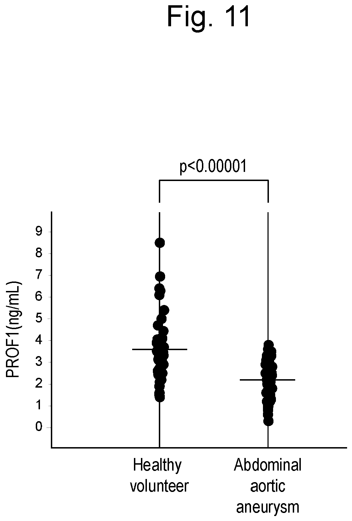

The results of PROF1 are shown in Figure 11.

From the results shown in Figure 11, the serum PROF1 values were 3.588±1.508 ng/mL in healthy subject (44 cases) and 2.193±0.910 ng/mL in abdominal aortic aneurysm patients (41 cases), respectively. Thus, the serum PROF1 value was significantly decreased in the abdominal aortic aneurysm patients. Therefore, PROF1 was demonstrated to be a useful abdominal aortic aneurysm marker.

### - Result 2 (Evaluation of PROF1 as abdominal aortic aneurysm marker based on ROC curves)

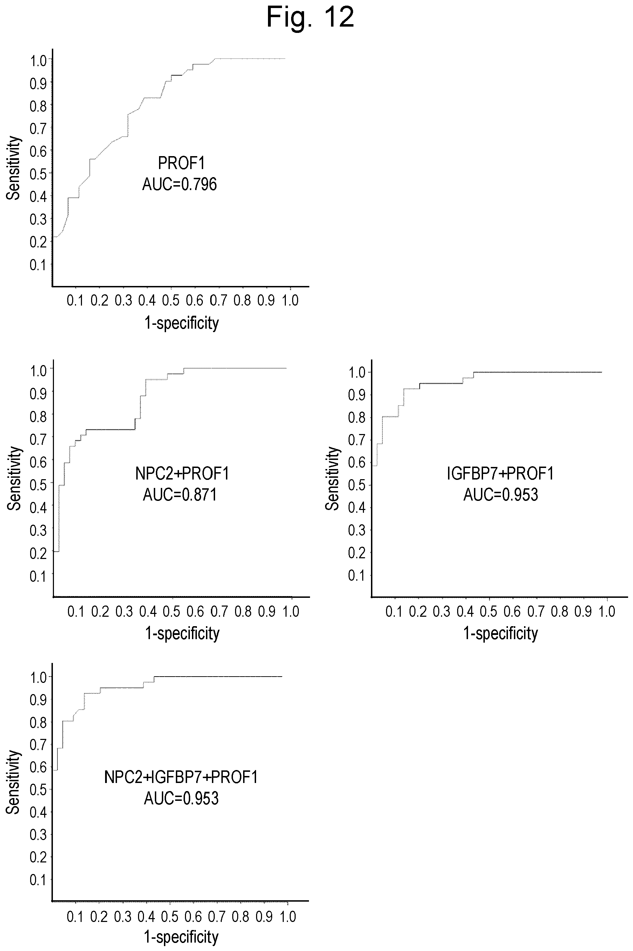

The ROC (Receiver Operating Characteristic) curves of PROF1 in the diagnosis of abdominal aortic aneurysm are shown in Figure 12. The ROC curves were produced using statistical analysis software StatFlex Ver. 6.0 (Artec Co., Ltd.). The AUC (Area Under the Curve) value of PROF1 obtained from the ROC curve was 0.796. In addition, the AUC values were obtained from the ROC curve regarding the combinations of PROF1 with NPC2 and IGFBP7, which were measured by the method described in Example 4. As a result, the AUC value was 0.871 when PROF1 was combined with NPC2, and the AUC value was 0.953 when PROF1 was combined with IGFBP7. Also, the AUC value was 0.953 in the combination of PROF1, NPC2 and IGFBP7. Therefore, it was demonstrated that PROF1 is a useful abdominal aortic aneurysm marker, and that the usefulness is further increased by combining PROF1 with NPC2 and/or IGFBP7.

### [Comparative Example 1]

### Comparison 1 with conventional marker (CRP)

The CRP values of the serum specimens used in Example 1 were measured. As a measurement reagent, C-Reactive Protein Kit CRP-LATEX X2 "SEIKEN" NX (DENKA SEIKEN Co., Ltd.) was used, and as measurement devices, LABOSPECT 008 Hitachi Automated Analyzer and 7180-Type Hitachi Automated Analyzer (Hitachi High-Technologies Corporation) were used. The ROC curves were produced using statistical analysis software StatFlex Ver. 6.0 (Artec Co., Ltd.).

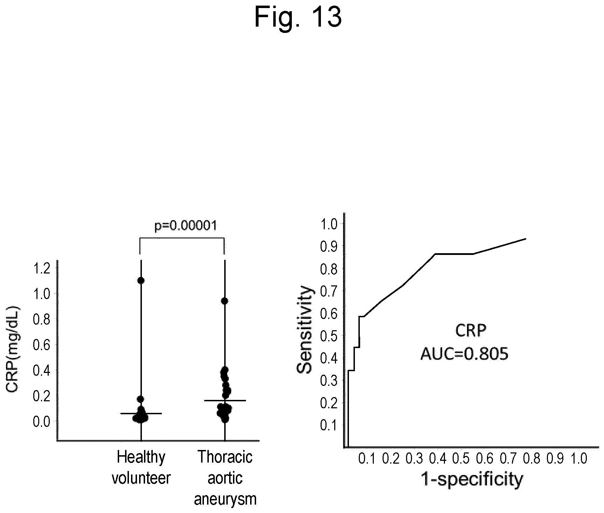

The results are shown in Figure 13.

The AUC (Area Under the Curve) value obtained from the ROC curve was 0.805. Therefore, it was demonstrated that both NPC2 (AUC = 0.947) and IGFBP7 (AUC = 0.930) as shown in Example 1 are thoracic aortic aneurysm markers that are more useful than CRP.

### [Comparative Example 2]

### Comparison 2 with conventional marker (CRP)

The CRP values of the serum specimens used in Example 4 were measured. As a measurement reagent, C-Reactive Protein Kit CRP-LATEX X2 "SEIKEN" NX (DENKA SEIKEN Co., Ltd.) was used, and as measurement devices, LABOSPECT 008 Hitachi Automated Analyzer and 7180-Type Hitachi Automated Analyzer (Hitachi High-Technologies Corporation) were used. The ROC curves were produced using statistical analysis software StatFlex Ver. 6.0 (Artec Co., Ltd.).

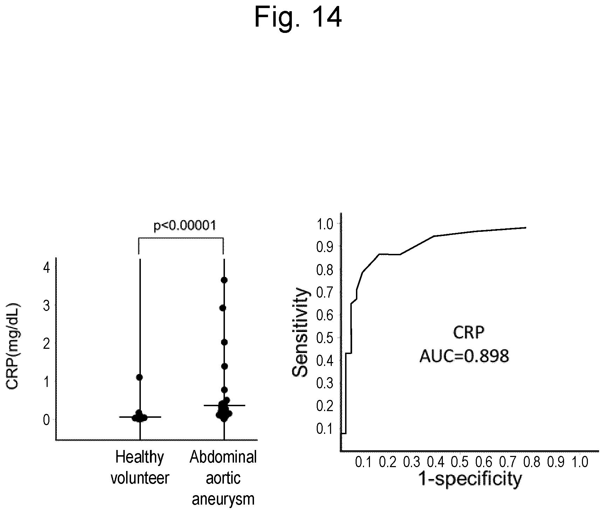

The results are shown in Figure 14.

The AUC (Area Under the Curve) value obtained from the ROC curve was 0.898. Therefore, it was demonstrated that all of the combination of NPC2 and IGFBP7 (Example 4: AUC = 0.910), the combination of IGFBP7 and TSP1 (Example 5: AUC = 0.909), the combination of NPC2, IGFBP7 and TSP1 (Example 5: AUC = 0.915), the combination of IGFBP7 and PROF1 (Example 6: AUC = 0.953), and the combination of NPC2, IGFBP7 and PROF1 (Example 6: AUC = 0.953) are abdominal aortic aneurysm markers that are more useful than CRP.

### [Example 7]

### Comparison 1 with other diseases (Measurement of IGFBP7 in serum specimens according to ELISA method)

The serum concentration of IGFBP7 was measured in patients with other diseases (5 cases of colorectal cancer, 5 cases of stomach cancer, 5 cases of non-small cell lung cancer, and 4 cases of hepatocellular carcinoma) and in thoracic aortic aneurysm patients (29 cases) according to ELISA. The serum concentration of IGFBP7 was measured in accordance with the method described in Example 1. For the statistical treatment performed to compare the other disease patient group with the thoracic aortic aneurysm patient group, StatFlex Ver. 6.0 (Artec Co., Ltd.) was used. For the significance test, Mann-Whitney U Test was used.

### - Results

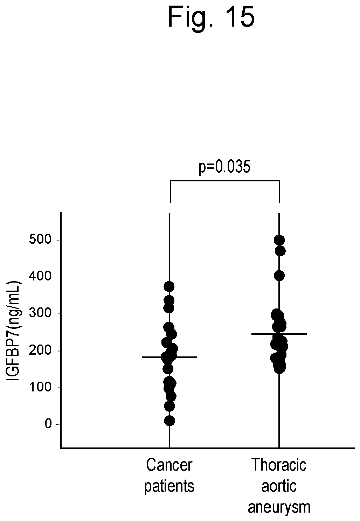

The results are shown in Figure 15.

From the results shown in Figure 15, the serum IGFBP7 values were 180.632±97.397 ng/mL in the patients with other diseases (a total of 19 cases) and 243.881±96.857 ng/mL in thoracic aortic aneurysm patients (29 cases), respectively. Thus, the serum IGFBP7 value was significantly increased in the thoracic aortic aneurysm patients. Therefore, it was demonstrated that IGFBP7 would be likely to become a diagnostic marker of thoracic aortic aneurysm, which can distinguish thoracic aortic aneurysm from diseases other than cardiovascular diseases.

### [Example 8]

### Comparison 2 with other diseases (Measurement of IGFBP7 in serum specimens according to ELISA method)

The serum concentration of IGFBP7 was measured in patients with other diseases (5 cases of colorectal cancer, 5 cases of stomach cancer, 5 cases of non-small cell lung cancer, and 4 cases of hepatocellular carcinoma) and in abdominal aortic aneurysm patients (51 cases) according to ELISA. The serum concentration of IGFBP7 was measured in accordance with the method described in Example 1. For the statistical treatment performed to compare the other disease patient group with the abdominal aortic aneurysm patient group, StatFlex Ver. 6.0 (Artec Co., Ltd.) was used. For the significance test, Mann-Whitney U Test was used.

### - Results

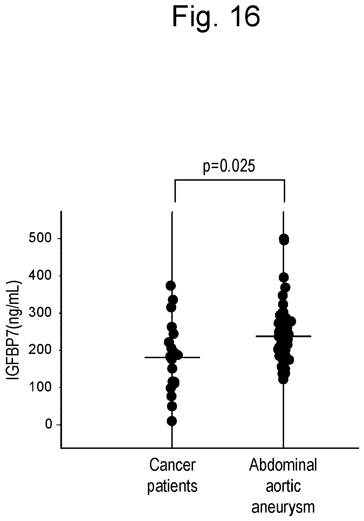

The results are shown in Figure 16.

From the results shown in Figure 16, the serum IGFBP7 values were 180.632±97.397 ng/mL in the patients with other diseases (a total of 19 cases) and 236.588±82.702 ng/mL in abdominal aortic aneurysm patients (51 cases), respectively. Thus, the serum IGFBP7 value was significantly increased in the abdominal aortic aneurysm patients. Therefore, it was demonstrated that IGFBP7 would be likely to become a diagnostic marker of abdominal aortic aneurysm, which can distinguish abdominal aortic aneurysm from diseases other than cardiovascular diseases.

### Industrial Applicability

By using NPC2 and/or IGFBP7 that are the novel marker(s) of aortic aneurysm according to the present invention, the onset of aortic aneurysm can be specifically detected with high sensitivity. Therefore, the agent and kit for detecting aortic aneurysm according to the present invention can be used in the screening of aortic aneurysm, assistance for the early diagnosis of aortic aneurysm, the development and evaluation of a preventive or therapeutic agent therefor, and the like. Further, the present invention can be utilized in the industry for producing the detection agent and the detection kit.

All publications, patents and patent applications cited in the present description are incorporated herein by reference in their entirety.

SEQUENCE LISTING

<110>  EIKEN KAGAKU KABUSHIKI KAISHA
       NATIONAL CEREBRAL AND CARDIOVASCULAR CENTER

<120>  A NOVEL MARKER FOR AORTIC ANEURYSM

<130>  PH-7839-PCT

<150>  PCT/JP2018/013571
<151>  2018-03-30

<160>  4

<170>  PatentIn version 3.5

<210>  1
<211>  151
<212>  PRT
<213>  Homo sapiens

<400>  1

<210>  2
<211>  282
<212>  PRT
<213>  Homo sapiens

<400>  2

Met Glu Arg Pro Ser Leu Arg Ala Leu Leu Leu Gly Ala Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60

Arg Gly Glu Gly Glu Pro Cys Gly Gly Gly Gly Ala Gly Arg Gly Tyr
65                  70                  75                  80

Cys Ala Pro Gly Met Glu Cys Val Lys Ser Arg Lys Arg Arg Lys Gly
                85                  90                  95

Lys Ala Gly Ala Ala Ala Gly Gly Pro Gly Val Ser Gly Val Cys Val
            100                 105                 110

Cys Lys Ser Arg Tyr Pro Val Cys Gly Ser Asp Gly Thr Thr Tyr Pro
        115                 120                 125

Ser Gly Cys Gln Leu Arg Ala Ala Ser Gln Arg Ala Glu Ser Arg Gly
    130                 135                 140

Glu Lys Ala Ile Thr Gln Val Ser Lys Gly Thr Cys Glu Gln Gly Pro
145                 150                 155                 160

Ser Ile Val Thr Pro Pro Lys Asp Ile Trp Asn Val Thr Gly Ala Gln
                165                 170                 175

Val Tyr Leu Ser Cys Glu Val Ile Gly Ile Pro Thr Pro Val Leu Ile
            180                 185                 190

Trp Asn Lys Val Lys Arg Gly His Tyr Gly Val Gln Arg Thr Glu Leu
        195                 200                 205

Leu Pro Gly Asp Arg Asp Asn Leu Ala Ile Gln Thr Arg Gly Gly Pro
    210                 215                 220

Glu Lys His Glu Val Thr Gly Trp Val Leu Val Ser Pro Leu Ser Lys
225                 230                 235                 240

Glu Asp Ala Gly Glu Tyr Glu Cys His Ala Ser Asn Ser Gln Gly Gln
                245                 250                 255

Ala Ser Ala Ser Ala Lys Ile Thr Val Val Asp Ala Leu His Glu Ile
            260                 265                 270

Pro Val Lys Lys Gly Glu Gly Ala Glu Leu
        275                 280

<210>  3
<211>  1170
<212>  PRT
<213>  Homo sapiens

<400>  3

<210> 4
<211> 140
<212> PRT
<213> Homo sapiens

<400> 4

## Claims

1. A method for detecting aortic aneurysm, comprising a step of measuring the following 1) and/or 2) in a sample obtained from a subject:
1) an NPC2 (Niemann-Pick disease type C2) protein, and/or
2) an IGFBP7 (Insulin-like growth factor-binding protein 7) protein.

2. The method according to claim 1, further comprising a step of measuring 3) a TSP1 (Thrombospondin 1) protein and/or 4) a PROF1 (Profilin 1) protein.

3. The method according to claim 1, wherein the subject is an animal in need of prevention or treatment of aortic aneurysm, and the effects of a preventive or therapeutic agent for aortic aneurysm are evaluated or determined by using the NPC2 protein and/or the IGFBP7 protein as indicator(s).

4. The method according to claim 2, wherein the subject is an animal in need of prevention or treatment of aortic aneurysm, and the effects of a preventive or therapeutic agent for aortic aneurysm are evaluated or determined by using the NPC2 protein and/or the IGFBP7 protein and the TSP1 protein and/or the PROF1 protein as indicators.

5. A method for detecting or screening a subject having a risk of developing aortic aneurysm in future, comprising a step of measuring the following 1) and/or 2) in a sample obtained from the subject:
1) an NPC2 (Niemann-Pick disease type C2) protein, and/or
2) an IGFBP7 (Insulin-like growth factor-binding protein 7) protein.

6. The method according to claim 5, further comprising a step of measuring 3) a TSP1 (Thrombospondin 1) protein and/or 4) a PROF1 (Profilin 1) protein.

7. The method according to any one of claims 1 to 6, wherein the aortic aneurysm is thoracic aortic aneurysm, thoracoabdominal aortic aneurysm, and/or abdominal aortic aneurysm.

8. The method according to any one of claims 1 to 7, wherein the sample is selected from the group consisting of serum, plasma and blood.

9. The method according to any one of claims 1 to 8, wherein the subject is a human.

10. The method according to any one of claims 1 to 9, wherein the measurement method is immunoassay or mass spectrometry.

11. A detection marker of aortic aneurysm, comprising the following 1) and/or 2):
1) an NPC2 protein, and/or
2) an IGFBP7protein.

12. The detection marker according to claim 11, further comprising 3) a TSP1 protein and/or 4) a PROF1 protein.

13. The detection marker according to claim 11 or 12, wherein the aortic aneurysm is thoracic aortic aneurysm, thoracoabdominal aortic aneurysm, and/or abdominal aortic aneurysm.

14. An agent for detecting aortic aneurysm, comprising an antibody or an aptamer for measuring the detection marker according to any one of claims 11 to 13.

15. The detection agent according to claim 14, wherein the antibody is a monoclonal antibody and/or a polyclonal antibody.

16. A kit for detecting aortic aneurysm, comprising the detection agent according to claim 14 or 15.

17. The detection marker, detection agent or detection kit according to any one of claims 11 to 16, wherein the sample used in detection of aortic aneurysm is selected from the group consisting of serum, plasma and blood.

18. Use of the detection marker according to any one of claims 11 to 13, which is for use in screening a preventive or therapeutic agent for aortic aneurysm.

19. Use of the detection marker according to any one of claims 11 to 13, which is for use in detecting or screening *in vitro* a subject having a risk of developing aortic aneurysm in future.
